# EUROPÄISCHE PATENTSCHRIFT

(11) **EP 1 013 306 B1**
(45) Veröffentlichungstag und Bekanntmachung des Hinweises auf die Patenterteilung: **06.04.2005**
(21) Anmeldenummer: 99250396.1
(22) Anmeldetag: 06.11.1999
(51) Int. Cl.: A61N 1/368

(54) **Zweikammer-Herzschrittmacher**
Dual chamber pacemaker
Stimulateur cardiaque double chambre

(30) Priorität: 15.12.1998 DE 19859651
(43) Veröffentlichungstag der Anmeldung: 28.06.2000
(73) Patentinhaber: BIOTRONIK Mess- und Therapiegeräte GmbH & Co Ingenieurbüro Berlin, 12359 Berlin (DE)
(72) Erfinder: Backers, Jos, 8460 Oudenburg (BE)
(74) Vertreter: Eisenführ, Speiser & Partner

(56) Entgegenhaltungen:
- EP-A- 0 488 840
- EP-A- 0 677 303
- EP-A- 0 714 676
- EP-A- 0 755 696
- EP-A- 0 845 282
- WO-A-97/43002
- US-A- 4 467 810

## Beschreibung

Die Erfindung betrifft einen Zweikammer-Herzschrittmacher gemäß dem Oberbegriff des Anspruchs 1.

Zweikammer-Herzschrittmacher, die den Ventrikel synchron zu atrialen Ereignissen stimulieren, können eine Betriebsartumschaltungs("Mode-Switch")-Funktion aufweisen, die darin besteht, daß bei Überschreiten einer oberen Grenzrate wahrgenommener atrialer Herzaktionen von der atriumsynchronen in eine asynchrone Betriebsart umgeschaltet wird.

Der umgekehrte Vorgang, nämlich der Wechsel von einer asynchronen in eine atriumsynchrone Betriebsart, also das Beenden von Mode-Switch-Episoden, wird vom Herzschrittmacher normalerweise aufgrund des sogenannten "x aus y-Kriteriums", d.h. nach einer bestimmten Anzahl atrial wahrgenommener Ereignisse mit einer Rate unterhalb der oberen atrialen Grenzrate innerhalb einer vorgegebenen Gesamtzahl atrialer Ereignisse, ausgelöst. Bei der auf die Zurückschaltung in den atriumsynchronen Betrieb folgenden Resynchronisation der ventrikulären Stimulationsimpulse auf atrial wahrgenommene Ereignisse können grundsätzlich zwei erhebliche Fehler auftreten:

Erstens können die atrial wahrgenommenen Ereignisse aus der retrograden Überleitung ventrikulärer Stimulationsimpulse resultieren. Dabei verursacht jedes ventrikulär stimulierte Ereignis ein retrograd übergeleitetes atriales Ereignis, das wiederum eine AV-Zeit auslöst, nach der ein ventrikuläres Ereignis stimuliert wird, das wieder ein retrograd übergeleitetes atriales Ereignis verursacht etc.

Die Resynchronisation auf die atrial wahrgenommenen Ereignisse würde dann eine Synchronisation auf die Ventrikel-Stimuli bedeuten und unmittelbar eine schrittmacherinduzierte Tachykardie (PMT - "pacemaker mediated tachycardia") auslösen.

Zweitens kann die atrial wahrgenommene Rate aus einer 2:1-Wahrnehmung atrialer Ereignisse aufgrund des atrialen Blanking resultieren, das jedes zweite atriale Ereignis für den Schrittmacher "unsichtbar" macht. Die Resynchronisation würde dann unmittelbar zum 2:1-Block-Verhalten führen, das physiologisch natürlich höchst unerwünscht ist.

Die US 5,549,648 beschreibt ein Schrittmachersystem und Betriebsverfahren zur verbesserten Erfassung des Endes einer Phase retrograder Überleitung. Sie beschäftigt sich mit einer möglichst schnellen Erkennung des Zustands, in dem die Mode-Switch-Episode beendet werden kann. Das Ziel dieser Druckschrift ist eine Analyse der atrialen sowie der ventrikulären Herzaktionen auf einer Schlag-zu-Schlag-Basis anhand bestimmter Kriterien ("beat-to-beat"-Analyse). Aufgrund des Ergebnisses dieser Analyse soll dann der Schrittmacherbetrieb resynchronisiert werden.

Die obengenannten Fehler, die bei der Resynchronisation auftreten können, werden bei dem in dieser Druckschrift beschriebenen Vorgehen nicht beseitigt. Im Gegenteil macht deren Zielsetzung, die Resynchronisation beschleunigt einzuleiten, die Überprüfung des Analysenergebnisses zur Ausschaltung dieser Fehler tendenziell unmöglich.

Der Erfindung liegt deshalb die Aufgabe zugrunde, einen Zweikammerschrittmacher anzugeben, bei dem eine Fehlfunktionen weitgehend ausschließende Zurückschaltung aus einer asynchronen in eine atriumsynchrone Betriebsart und Resynchronisation auf das Atrium möglich ist.

Die Aufgabe wird durch einen Zweikammerschrittmacher mit den im Anspruch 1 angegebenen Merkmalen gelöst.

Um ein mögliches Fehlverhalten des Schrittmachers bei der Zurückschaltung und Resynchronisation zu vermeiden, wird für die Erkennung des Zustands, in dem die Mode-Switch-Episode beendet werden kann, folgende Funktionsweise vorgeschlagen:
a) Ein zum Nachweis der Erfüllung des oben angesprochenen x-aus-y-Kriteriums eingesetzter Zähler ("x-aus-y-Zähler") wird nur bei Auftreten bestimmter bekannter Folgen von Herzereignissen (nachfolgend auch als "Signalfolgemuster" bezeichnet) inkrementiert.
Dies sind insbesondere die nachstehenden Ereignisfolgen:

| | |
|---|---|
| As-V-As wobei As-As | > 1.000 ms |
| V-As-As-V wobei As-As | > 1.000 ms |
| V-V-As wobei V-As | > 1.000 ms |
| V-V ohne dazwischenliegendes As As = atrial wahrgenommenes Ereignis, V = ventrikulär wahrgenommenes oder stimuliertes Ereignis) | |

b) Erfüllt der Zählerstand ein vorbestimmtes x-aus-y-Kriterium, wird - insbesondere in Form einer Modulation bzw. Variation mehrerer V-V-Intervalle - ein Test auf das Vorliegen einer retrograden Überleitung durchgeführt. Weisen die auf die Modulationen jeweils folgenden V-As-Intervalle eine konstante Dauer auf, liegt eine retrograde Überleitung vor, ist dagegen der Zeitpunkt, zu dem atrial wahrgenommene Ereignisse auftreten, unabhängig vom (variierten) Zeitpunkt der ventrikulären Stimulation, waren die Ereignisse nicht retrograd übergeleitet.

Die Modulation wird zweckmäßigerweise als Verlängerung der Zeitabstände um eine postventrikuläre atriale Austastzeit und ein zusätzliches Inkrement ausgeführt und die Resynchronisation aktiviert, wenn mindestens drei aufeinanderfolgende VA-Zeitintervalle nach modulierten Ventrikelstimuli konstant waren.

Die zusätzliche Überprüfung erfordert gegenüber dem herkömmlichen x-aus-y-Ratenkriterium allenfalls einen zusätzlichen Testzyklus, bevor (bei positivem Ausgang der Tests) resynchronisiert werden kann, erhöht aber wesentlich die Sicherheit vor einer unangebrachten und die Gefahr einer PMT heraufbeschwörenden Zurückschaltung in den synchronen Betrieb.

In einer weiteren vorteilhaften Ausgestaltung der Erfindung ist vorgesehen, daß der Kriterienspeicher zur Speicherung mindestens eines die Zeitintervalle zwischen aufeinanderfolgenden atrialen Aktivitäten betreffenden Raten-Schwellwertes - bevorzugt zur Speicherung zweier verschiedener Schwellwerte in verschiedenen Stufen des Testablaufes - ausgebildet ist und die Verarbeitungseinheit eine Berechnungseinheit zur Ermittlung der aktuellen atrialen Zeitintervalle bzw. atrialen Rate und (mindestens) eine Ratenvergleichereinheit zu deren Vergleich mit dem gespeicherten Raten-Schwellwert aufweist.

Das Ergebnis des Ratenvergleiches wird zusammen mit dem Ergebnis der x-aus-y-Zählung einer Logikstufe zugeführt. Eine Aktivierung der Schaltmittel zur Umschaltung in den synchronen Modus und Resynchronisation der Ventrikelstimulation wird im Ergebnis der logischen Verarbeitung - Erreichen des Zählerstandes "x" durch die Zählermittel vorausgesetzt - vom zusätzlichen Vorliegen eines Ausgangssignals der Ratenvergleichereinheit abhängig gemacht, das die Unterschreitung eines vorbestimmten Schwellwertes der atrialen Rate ("Entscheidungsrate") anzeigt.

Die Logikstufe realisiert nach obigem eine UND-Verknüpfung der x-aus-y-Zählung und des zusätzlichen Tests durch Modulation des VV-Stimulationsintervalls, der insbesondere noch eine ODER-Verknüpfung zwischen dem Testergebnis und dem zuletzt erwähnten Ratenvergleich vorgeschaltet ist.

Die Stimulations-Steuereinheit realisiert zweckmäßigerweise eine - als solche bekannte - dynamische Ratenbegrenzung der ventrikulären Stimulationsrate zur Vermeidung einer PMT ("dynamic PMT limit"). Im Zusammenhang mitderErfindung wird diese Funktion dahingehend weiterentwickelt, daß der konkrete Ratengrenzwert in Abhängigkeit vom Ergebnis der vorgesehenen Testschritte ausgewählt wird:

Wird im Ergebnis der Tests der Schrittmacherbetrieb auf atrialen Sinusrhythmus (spontane atriale Rate > sensorindizierte Rate) eingestellt, sollte die Ratenbegrenzung auf der Rate der spontanen atrialen Aktivität ("Sinusrate") basieren. Mündet die vorangegangene Tachykardie aber in atriale Bradykardie (spontane atriale Rate < sensorindizierte Rate), sollte die Grenzrate aus der sensorindizierten Rate abgeleitet werden. Dies gilt auch für den Fall, daß retrograde Überleitung festgestellt und eine aktive Resynchronisation durchgeführt wurde.

Vorteilhafte Weiterbildungen der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1 ein Funktions-Blockschaltbild eines Zweikammer-Schrittmachers gemäß einer bevorzugten Ausführungsform der Erfindung,
- Figur 2 ein Flußdiagramm zum Betrieb des Zweikammer-Schrittmachers nach Fig. 1,
- Figur 2a eine Subroutine des Flußdiagramm nach Fig. 2,
- Figur 3 ein Timingdiagramm zu einem Schritt einer bevorzugten Testprozedur und
- Figur 4 ein Funktions-Blockschaltbild einer bevorzugten Ausführung der Stimulations-Steuereinheit des Schrittmachers nach Fig. 1.

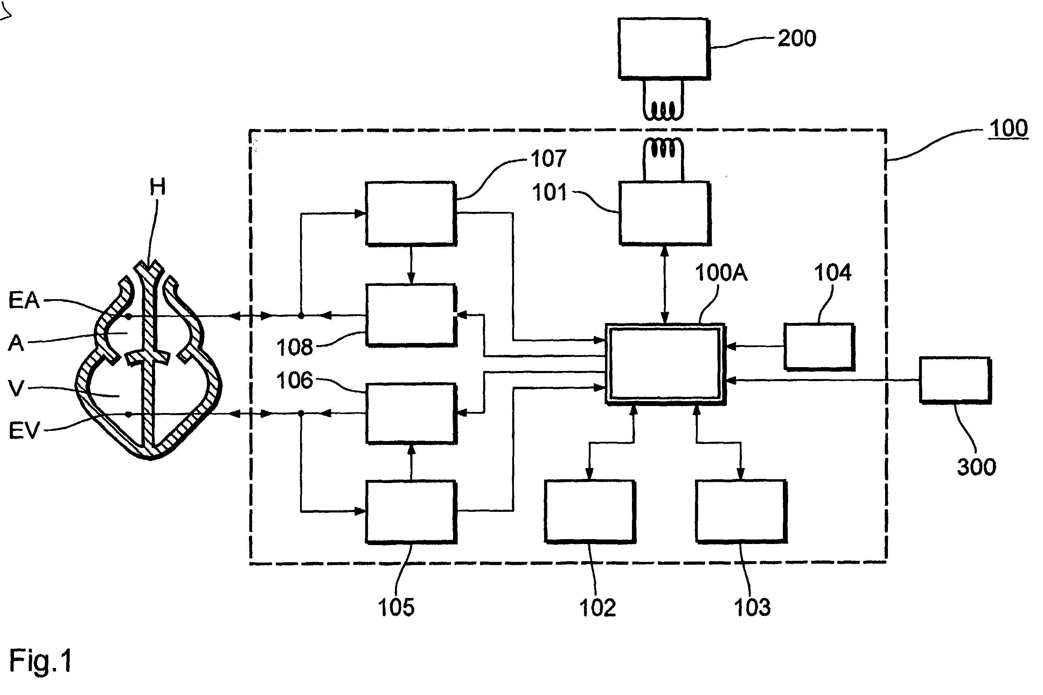

Fig. 1 zeigt in einer Prinzpdarstellung die wesentlichen Komponenten einer aus einem Zweikammer-Herzschrittmacher 100, einem Programmiergerät 200, einem Körpersensor 300 sowie einer Atriumelektrode EA im Atrium A und einer Ventrikelektrode EV im Ventrikel V eines Herzens H bestehenden Schrittmacheranordnung. Zur Verbindung des implantierten Schrittmachers 100 mit dem Programmiergerät 200 ist eine interne Telemetrieeinheit 101 und zur Betriebssteuerung eine Stimulations-Steuereinheit 100A vorgesehen.

Letzterer sind in üblicher Weise ein interner Programmspeicher 102 und ein Datenspeicher 103 sowie ein Takt- und Zeitgeber 104 zugeordnet. Der Körpersensor 300 - in an sich bekannten Ausführungen beispielsweise ein piezoelektrischer Aktivitätsfühler, ein Sensor für die Blutsauerstoffsättigung oder Bluttemperatur oder ein Impedanzplethysmograph - ist mit einem Eingang der Stimulations-Steuereinheit 100A verbunden. Mit der Ventrikelelektrode EV ist eingangsseitig eine Ventrikel-Abfühleinheit 105 und ausgangsseitig eine Ventrikel-Stimulationseinheit 106 verbunden, und analog ist mit der Atriumelektrode EA eingangsseitig eine Atrium-Abfühleinheit 107 und ausgangsseitig eine Atrium-Stimulationseinheit 108 verbunden. Die Abfühleinheiten 105, 107 sind mit Dateneingängen der Stimulations-Steuereinheit 100A verbunden, und die Stimulationseinheiten 106, 108 sind mit Steuersignalausgängen derselben verbunden.

Der Zweikammer-Schrittmacher 100 ist zu einem ratenadaptiven Zweikammer-Demandbetrieb aufgrund von Ratensteuersignalen ausgebildet, die - auf an sich bekannte Weise aufgrund eines programmierten und ggfs. über das Programmiergerät veränderbaren Algorithmus - aufgrund der Signale des Körpersensors 300 in der Stimulations-Steuereinheit 1 00A gebildet und den Stimulationseinheiten 106, 108 zugeführt werden. Er ist weiterhin (in ebenfalls bekannter Weise) zu einer Umschaltung zwischen einem atriumsynchronen Betrieb in einen asynchronen Betrieb während Episoden von Vorhofflattern und zur Rückschaltung in den synchronen Betrieb bei Beendigung solcher Episoden ausgebildet. Die Besonderheit der vorgeschlagenen Lösung liegt in einem die Anwendung spezieller Kriterien bzw. Tests im Zusammenhang mit der Zurückschaltung ermöglichenden Aufbau und Betrieb.

Grundsätzlich wird die Zurückschaltung vorbereitet, wenn für eine vorbestimmte Anzahl von Herzzyklen aus einer ebenfalls vorbestimmten Gesamtzahl eines der weiter oben aufgelisteten Signalfolgemuster vorliegt, die als Indizien für die Beendigung der atrialen Tachykardie anzusehen sind.

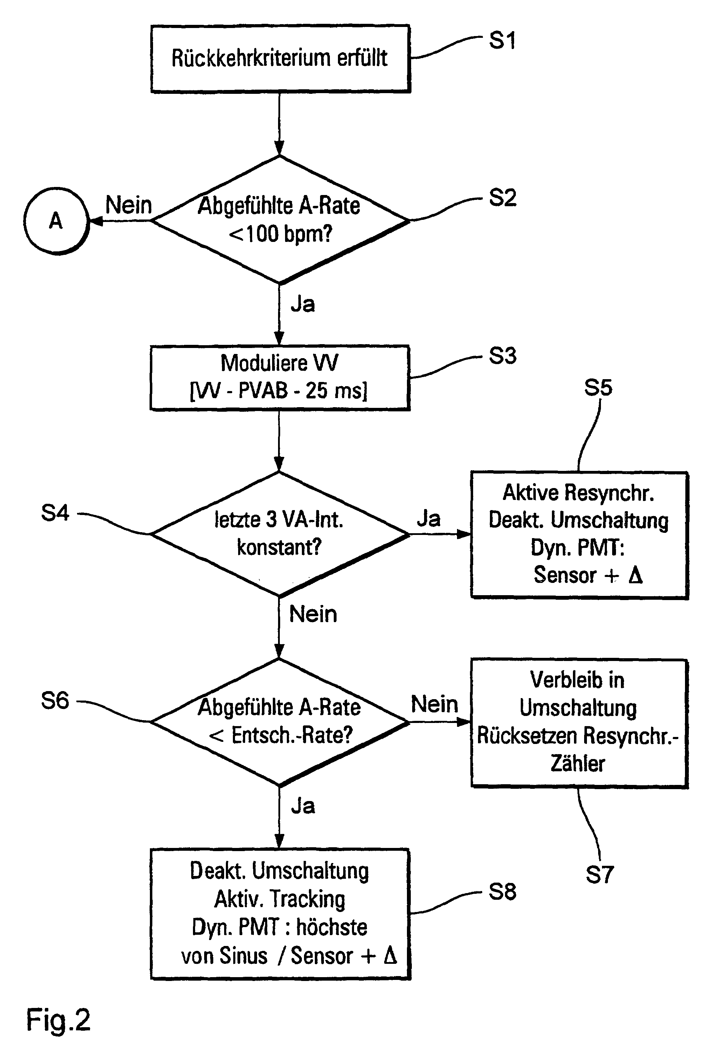

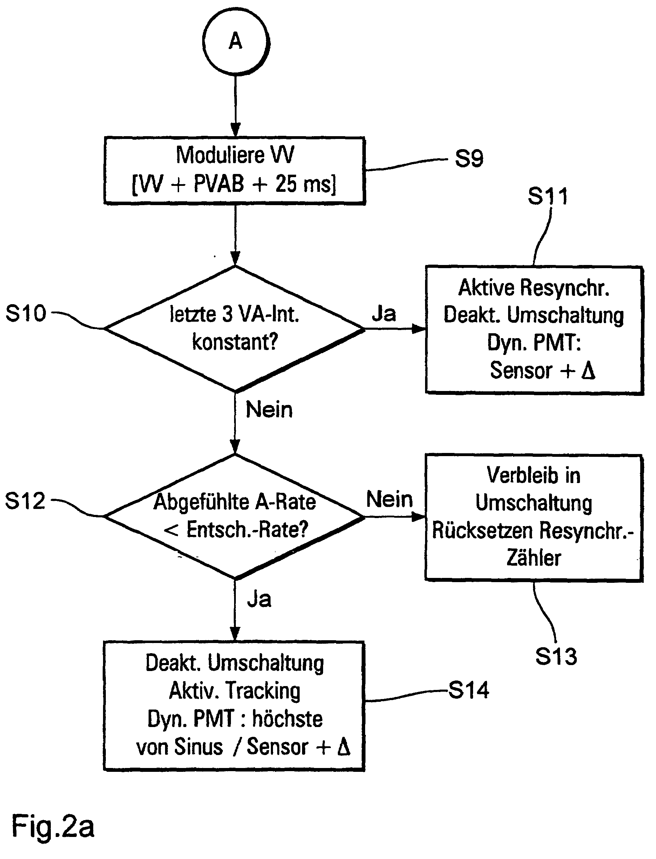

In Fig. 2, die ein Flußdiagramm zum Betrieb des Zweikammer-Schrittmachers 100 darstellt, ist dieser Zustand im Schritt S1 erreicht. Daraufhin wird in einem Schritt S2 festgestellt, ob die atriale Rate unterhalb eines ersten Raten-Grenzwertes (hier 100 bpm) liegt. Ist dies nicht der Fall, wird am Verzweigungspunkt "A" zur in Fig. 2a gezeigten Subroutine übergegangen. Ist die Bedingung hingegen erfüllt, wird in einem Schritt S3 für eine vorbestimmte Anzahl von Ventrikel-Stimuli deren Abstandsintervall bzw. Rate geändert, und zwar wird das VV-Intervall im vorliegenden Beispiel um die postventrikuläre atriale Austastperiode PVAB und zusätzlich um 25 ms reduziert. In einem Schritt S4 wird ermittelt, ob nach der Variation des VV-Intervalls drei VA-Intervalle konstant geblieben sind.

Trifft dies zu, so war die erfaßte atriale Rate auf retrograde Überleitung zurückzuführen. Der aufgrund des Vorhofflatterns eingenommene Umschaltzustand wird in einem Schritt S5 deaktiviert und der Schrittmacher aktiv auf die atriale Aktivität resynchronisiert. Dabei wird eine dynamische Ratenbegrenzung (abgekürzt als "Dyn. PMT") vorgenommen, die sich an der sensorindizierten Rate orientiert.

Sind hingegen die VA-Intervalle nicht konstant geblieben, so wird in einem weiteren Schritt S6 die erfaßte atriale Rate mit einem weiteren Grenzwert ("Entscheidungsrate" - gemäß den weiter oben aufgelisteten Kriterien 60 bpm) verglichen. Ist sie gleich diesem oder höher, ist davon auszugehen, daß beim vorangehenden Test eine 2:1-Erfassung einer tatsächlich oberhalb der Entscheidungsrate liegenden atrialen Rate vorgelegen hat. Deshalb wird in einem Schritt S7 der Verbleib im asynchronen Umschaltzustand entschieden und zugleich der Umschalt- bzw. Resynchronisationszähler auf Null zurückgesetzt.

Liegt die erfaßte Rate hingegen unter dem zweiten Grenzwert, kann in einem Schritt S8 der Umschaltzustand verlassen und in den synchronen Betrieb zurückgekehrt werden, wobei die dynamische ventrikuläre Ratenbegrenzung sich an der höheren Rate aus Sinusrate und sensorindizierter Rate orientiert.

Wie Fig. 2a zeigt, wird ausgehend vom Feld "A" in Fig. 2 in den Schritten S9 bis S14 eine dem Ablauf nach Fig. 2 sehr ähnliche Subroutine abgearbeitet, die jedoch im Schritt S9 mit einer andersartigen Variation der VV-Intervalle startet, nämlich mit einer Verlängerung um die PVAB plus 25 ms. Die Schritte S10 bis S14 stimmen mit den oben erläuterten Schritten S4 bis S8 überein und werden hier nicht nochmals beschrieben.

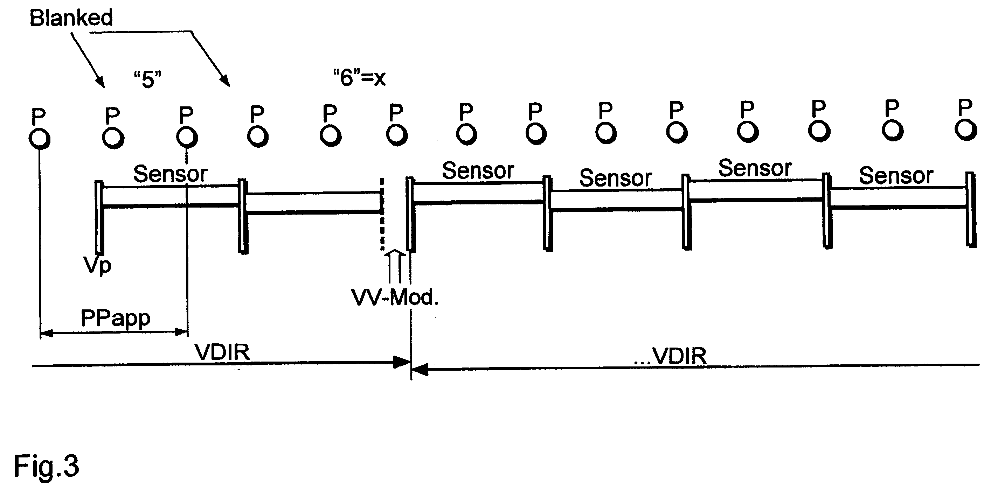

In Fig. 3 ist in Form eines Timingdiagramms eine vorteilhafte Auswirkung des oben beschriebenen Vorgehens gezeigt: Im linken Teil des Diagramms ist die Endphase eines x-aus-y-Tests zur Zurückschaltung aus dem asynchronen Betrieb (VDIR) in den synchronen Betrieb gezeigt, in der die Zählwerte 5 und 6 der Herzzyklen erreicht werden, in denen scheinbar ein Rückschaltkriterium (atriale Rate unterhalb der Entscheidungsrate) erfüllt ist. In Wirklichkeit wird jede zweite der atrialen Aktivitäten P kurz nach dem Ventrikelstimulus Vp ausgetastet ("Blanked"), so daß die von der atrialen Abfühleinheit "gesehenen" Aktionen aufgrund der 2:1-Wahrnehmung einen scheinbaren Abstand PPapp haben, der die Beendigung des Vorhofflatterns vortäuscht.

Nach Erreichung des als "x" vorbestimmten Zählerstandes 6 wird jedoch nicht sofort resynchronisiert, sondern zunächst der oben erläuterte VV-Modulationstest vorgenommen und - da dieser (angenommenermaßen) das Fortbestehen der atrialen Tachykardie ergibt - weiter in der VDIR-Betriebsart stimuliert. Damit wird ein 2:1-Blockverhalten vermieden, das sich ohne den zusätzlichen Test zwangsläufig einstellen würde.

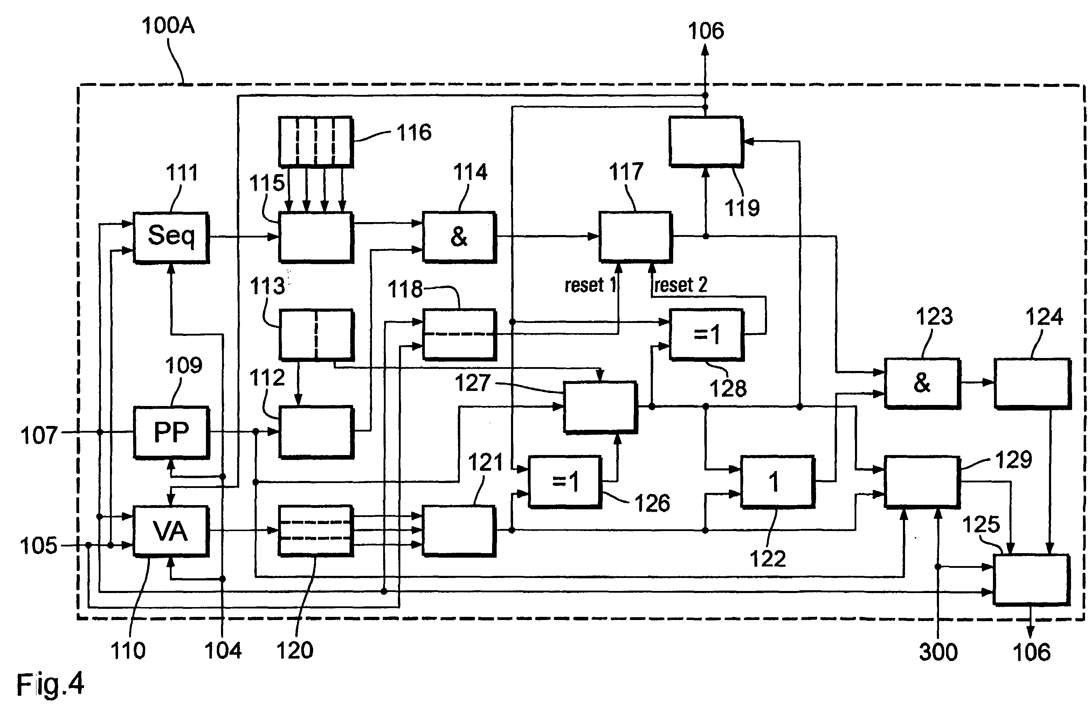

Fig. 4 zeigt in Form eines schematischen Funktions-Blockschaltbildes den - im Interesse eines leichteren Verständnisses vereinfachten - Aufbau der Stimulations-Steuereinheit 100A in einer Ausführungsform der Erfindung. Die der Ausführung der Erfindung dienenden Funktionskomponenten sind in der Praxis mindestens teilweise softwaremäßig realisiert und untrennbar mit der übrigen Schrittmacherstruktur verflochten.

Sie umfaßt eine mit dem Ausgang der Atrium-Abfühleinheit 107 verbundene PP-Intervall- bzw. Ratenberechnungsstufe 109, eine mit den Ausgängen der Atrium-Abfühleinheit 107 und der Ventrikel-Abfühleinheit 105 verbundene VA-Intervallberechnungsstufe 110 sowie eine ebenfalls eingangsseitig mit den Abfühleinheiten 105, 107 verbundene Sequenz-Bestimmungsstufe 111 zur Registrierung der Abfolge der Herzaktivitäten im Atrium und Ventrikel. Die Stufen 109 bis 110 sind jeweils auch mit dem Zeitgeber 104 (Fig. 1) verbunden, der die für die Intervall- bzw. Ratenberechnungen und die Signalfolgebestimmung benötigten Zeitsignale liefert.

Mit dem Ausgang der PP-Intervallberechnungsstufe 109 ist eine Ratenvergleicherstufe 112 verbunden, deren zweiter Eingang mit dem ersten Speicherbereich eines Zwei-Bereichs-Grenzratenspeichers 113 verbunden ist und in der ein Vergleich der ermittelten aktuellen atrialen Rate mit einer ersten Grenzrate ("Entscheidungsrate") ausgeführt wird. In dessen Ergebnis erscheint bei Unterschreitung der Grenzrate ein Ausgangssignal, das einem Eingang eines UND-Gatters 114 zugeführt wird.

Der Ausgang der Sequenz-Bestimmungsstufe 111 ist mit einem Eingang einer Signalfolge-Vergleichereinheit 115 verbunden, deren zweiter Eingang mit einem Signalfolgemusterspeicher 116 verbunden ist und deren Ausgang zum zweiten Eingang des UND-Gatters 114 führt. Der Ausgang des UND-Gatters taktet einen x-Zähler 117. Dieser weist zwei Rücksetzeingänge auf, von denen der eine mit dem Ausgang einer y-Zählerstufe 118 verbunden ist, welche ihrerseits eingangsseitig mit den Abfühleinheiten 105 und 107 verbunden ist. Die Belegung des zweiten Rücksetzeingangs wird weiter unten beschrieben.

Mit den oben genannten Funktionsblöcken 109 und 111 bis 118 wird unter paralleler Anwendung eines PP-Ratenkriteriums und eines Signalfolgekriteriums entsprechend den ersten beiden der weiter oben konkret genannten Kriterien für die Beendigung einer atrialen Tachyarrhythmie (auf die die Erläuterung hier in vereinfachender Weise beschränkt werden soll) die sogenannte "x-aus-y-Zählung" realisiert. Bei jedem von y aufeinanderfolgenden, in der Zählerstufe 118 gezählten Herzzyklen wird die Übereinstimmung mit einem der im Speicher 116 vorgespeicherten Signalfolgemuster und die Unterschreitung der im Speicher 113 gespeicherten Entscheidungsrate geprüft und bei Erfüllung beider Kriterien der x-Zähler 117 inkrementiert. Sobald er den Wert x erreicht hat, wird ein das positive Ergebnis der Prüfung anzeigendes Ausgangssignal ausgegeben. Im skizzierten Ausführungsbeispiel wird vereinfachend angenommen, daß der x-Zähler 117 bei Ablauf des y-Zählers 118 vor Erreichung des Zählwertes x auf Null zurückgesetzt und ein neuer Prüfzyklus gestartet wird. In der Praxis wird aber eine gleitende x-aus-y-Zählung unter Verwerfung des jeweils "ältesten" Herzzyklus nach dem FIFO-Prinzip zweckmäßiger sein, weil sie eine schnellere Reaktion des Schrittmachers ermöglicht.

Der Ausgang des x-Zählers 117 ist mit einer Stimulations-Teststufe 119 zur Beeinflussung des Abstandes einer vorprogrammierten Anzahl von Ventrikelstimuli verbunden. Der genaue Modus der Beeinflussung wird über einen Steuereingang der Stufe 119 eingestellt (siehe dazu weiter unten). Der Ausgang der Stimulations-Teststufe ist - neben der Ventrikel-Stimulationseinheit 106 - mit einem Steuereingang der VA-Berechnungsstufe 110 verbunden, über den kurz vor der Einleitung des oben erwähnten Modulations-Tests die Ermittlung der aktuellen VA-Intervalle und deren Übernahme in einen VA-Intervallspeicher 120 getriggert wird. Dieser ist mit dem Eingang eines VA-Auswertungseinheit 121 verbunden, in der die während des Modulations-Tests gespeicherten VA-Intervalle auf Konstanz innerhalb einer vorprogrammierten Schwankungsbreite geprüft werden. Bei Feststellung von Konstanz der VA-Intervalle wird ein entsprechendes Ausgangssignal ("1") ausgegeben, anderenfalls bleibt der Ausgang auf "0".

Im ersteren Falle wird über ein der VA-Auswertungseinheit 121 nachgeschaltetes ODER-Gatter 122 und UND-Gatter 123, dessen zweiter Eingang mit dem Ausgang des x-Zählers 117 verbunden ist, ein Aktivierungssignal an eine Umschalt- und Resynchronisationsstufe 124 übermittelt, die ihrerseits eine V-Impuls-Timingstufe 125, die zudem eingangsseitig mit der Atrium-Abfühleinheit 107 und dem Körpersensor 300 verbunden ist, im atriumsynchronen Betrieb steuert.

Bei negativem Ausgang des Modulations-Tests hingegen wird durch ein Test-Beendet-Signal von der Stimulations-Teststufe 119 und das "O"-Signal am Ausgang der VA-Auswertungsstufe 121 über ein mit diesen Stufen verbundenes EXOR-Gatter 126 eine zweite PP-Ratenvergleichereinheit 127 aktiviert. Diese ist eingangsseitig mit der PP-Ratenberechnungsstufe 109 und dem zweiten Speicherbereich des Grenzratenspeichers 113 verbunden und prüft, ob die aktuelle atriale Rate unterhalb einer zweiten vorprogrammierten Grenzrate (nach Fig. 2: 100 bpm) liegt.

Sie ist mit dem zweiten Eingang des oben erwähnten ODER-Gatters 122 verbunden und bewirkt über dieses sowie das UND-Gatter 123 eine Aktivierung der Umschaltund Resynchronisationsstufe 124, wenn dieses zusätzliche Kriterium erfüllt ist. Außerdem ist die Stufe 127 mit einem weiteren EXOR-Gatter 128 verbunden, über das bei Vorliegen des Test-Beendet-Signals von der Stimulations-Teststufe 119 und negativem Ausgang der Ratenprüfung (Ausgang "0" der Stufe 127) - nach negativem Ausgang der vorherigen Prüfung auf VA-Konstanz - der x-Zähler 117 über dessen zweiten Reset-Eingang auf Null zurückgesetzt wird. Schließlich ist die zweite Ratenvergleicherstufe 127 ausgangsseitig auch mit einem Steuereingang der Stimulations-Teststufe 119 verbunden, über den das modulierte Intervall zwischen den Ventrikelstimuli in Abhängigkeit vom Ausgang der Ratenprüfung eingestellt wird; vgl. dazu die Spezifikation der Schritte S3 und S9 in Fig. 2 und 2a.

Die VA-Auswertungsstufe 121 und die zweite Ratenvergleicherstufe 127 sind schließlich mit Steuereingängen einer Ratenbegrenzerstufe 129 verbunden, die über weitere Eingänge mit dem Körpersensor 300 und dem Ausgang der PP-Ratenberechnungsstufe 109 verbunden ist und aufgrund der aktuellen atrialen Rate und der Signale des Körpersensors sowie in Abhängigkeit von den Testergebnissen (wie weiter oben erläutert wurde) eine Ratenbegrenzung für die ventrikuläre Stimulation realisiert.

Die Erfindung beschränkt sich in ihrer Ausführung nicht auf die vorstehend anegebenen bevorzugten Ausführungsbeispiele. Vielmehr ist eine Anzahl von Varianten möglich, welche von der dargestellten Lösung auch in anders gearteter Ausführung Gebrauch machen.

## Patentansprüche

1. Zweikammer-Herzschrittmacher (100) mit
- Atrium-Abfühlmitteln (EA, 107) zum Abfühlen elektrischer Aktivität im Vorhof (A) und Ventrikel-Abfühlmitteln (EV, 2105) zum Abfühlen elektrischer Aktivität in der Herzkammer (V) eines Herzens (H),
- einem Zeitgeber (104) zur Erfassung des Zeitpunktes des Auftretens der jeweiligen elektrischen Aktivität,
- Ventrikel-Stimulationsmitteln (106, EV) zur Erzeugung von Stimulationsimpulsen und deren Abgabe an den Ventrikel und
- einer Stimulations-Steuereinheit (1 00A) zur Zeitsteuerung der Erzeugung und Abgabe der Stimulationsimpulse, welche Schaltmittel (124) zum Umschalten von einer asynchronen in eine vorhofsynchrone Betriebsart, Kriterienspeichermitteln (113, 116) zur Speicherung mindestens eines Umschaltkriteriums für die Betriebsart-Umschaltung und eine Verarbeitungseinheit (109, 111, 112, 114, 115) zur Verarbeitung von Signalen der Atrium-Abfühlmittel und der Ventrikel-Abfühlmittel und des Zeitgebers zur Prüfung der Erfüllung des Umschaltkriteriums aufweist,
**dadurch gekennzeichnet, daß**
- die Kriterienspeichermittel einen Signalfolgespeicher (113, 116) zur Speicherung einer vorbestimmten Auswahl von eine elektrische Aktivität des Herzens widerspiegelnden Signalfolgemustern und die Verarbeitungseinheit Vergleichermittel (112, 115) zum Vergleich der durch die Atrium-Abfühlmittel und die Ventrikel-Abfühlmittel in mehreren aufeinanderfolgenden Herzzyklen erfaßten elektrischen Aktivität mit den gespeicherten Signalfolgemustern aufweist und
- die Stimulations-Steuereinheit weiter eingangsseitig mit den Vergleichermitteln verbundene Zählermittel (117) zum Zählen der Anzahl von Herzzyklen, in denen Übereinstimmung der erfaßten elektrischen Aktivität mit einem der vorgespeicherten Signalfolgemuster festgestellt wurde, innerhalb einer vorbestimmten Gesamtzahl aufeinanderfolgender Herzzyklen aufweist, wobei die Zählermittel mittelbar mit einem Steuereingang der Schaltmittel verbunden sind.

2. Zweikammer-Herzschrittmacher nach Anspruch 1, **dadurch gekennzeichnet, daß** mindestens ein Teil der vorgespeicherten Signalfolgemuster eine vorbestimmte Abfolge von Signalen der Atrium-Abfühlmittel (107) und der Ventrikel-Abfühlmittel (105) oder Ventrikel-Stimulationsmittel (106) und ein den Abstand zweier atrial abgefühlter Aktivitäten oder einer atrial abgefühlten zu einer ventrikulären Aktivität betreffendes Zeitintervallkriterium umfaßt.

3. Zweikammer-Herzschrittmacher nach Anspruch 2, **dadurch gekennzeichnet, daß** die folgenden Signalfolgemuster gespeichert sind:
| | | |
|---|---|---|
| As-V-As | wobei As-As | > 1.000 ms |
| V-As-As-V | wobei As-As | > 1.000 ms |
| V-V-As | wobei V-As | > 1.000 ms |
| V-V | ohne dazwischenliegendes As, | |
wobei gilt: As = atrial wahrgenommenes Ereignis und V = ventrikulär wahrgenommenes oder stimuliertes Ereignis.

4. Zweikammer-Herzschrittmacher nach einem der vorangehenden Ansprüche, **dadurch gekennzeichnet, daß** die Stimulations-Steuereinheit (100A) eine Testeinrichtung (119, 110, 120, 121) zur Durchführung und Auswertung eines aktiven Tests auf das Vorliegen retrograder Überleitung aufweist, deren Ausgang mit einem Eingang einer zwischen die Zählermittel (117) und die Schaltmittel (124) geschalteten Logikstufe (122, 123, 126, 128) verbunden ist, so daß eine Aktivierung der Schaltmittel bei Anliegen eines Ausgangssignals der Zählermittel vom zusätzlichem Vorliegen eines ein positives Testergebnis repräsentierenden Ausgangssignals der Testeinrichtung abhängt.

5. Zweikammer-Herzschrittmacher nach Anspruch 4, **dadurch gekennzeichnet, daß** die Testeinrichtung Modulationsmittel (119) zu einer vorgegebenen Modulation des Zeitabstandes aufeinander folgender Ventrikelstimuli, VA-Erfassungsmittel (110, 120) zur Erfassung und Speicherung einer Mehrzahl von VA-Zeitintervallen zwischen jeweils einem Ventrikelstimulus und einer nachfolgenden atrialen Aktivität und VA-Auswertungsmittel (121) zur vergleichenden Auswertung der VA-Zeitintervalle in Reaktion auf die Modulation des Zeitabstandes der Ventrikelstimuli aufweist.

6. Zweikammer-Herzschrittmacher nach Anspruch 5, **dadurch gekennzeichnet, daß** die Modulationsmittel (119) zu einer Verlängerung oder Verkürzung der Zeitabstände um einen vorgegebenen Betrag, insbesondere um eine postventrikuläre atriale Austastzeit und ein zusätzliches Inkrement, ausgebildet sind und die VA-Auswertungsmittel (121) ein Ausgangssignal nur abgeben, wenn mindestens drei aufeinanderfolgende VA-Zeitintervalle nach modulierten Ventrikelstimuli konstant waren.

7. Zweikammer-Herzschrittmacher nach einem dervorhergehenden Ansprüche, **dadurch gekennzeichnet, daß** die Kriterienspeichermittel (113, 116) zur Speicherung mindestens eines die Zeitintervalle zwischen aufeinanderfolgenden atrialen Aktivitäten betreffenden Ratengrenzwertes, insbesondere zweier Ratengrenzwerte, ausgebildet sind und die Verarbeitungseinheit eine Berechnungseinheit (109) zur Ermittlung der aktuellen atrialen Zeitintervalle bzw. atrialen Rate und eine Raten-Vergleichereinheit (112, 127) zu deren Vergleich mit dem gespeicherten Raten-Schwellwert aufweist.

8. Zweikammer-Herzschrittmacher nach Anspruch 7 und einem der Ansprüche 4 bis 6, **dadurch gekennzeichnet, daß** der Ausgang der Ratenvergleichereinheit (127) mit einem Eingang der Logikstufe (122, 123, 126, 128) verbunden ist derart, daß eine Aktivierung der Schaltmittel (124) bei Anliegen eines Ausgangssignals der Zählermittel (117) vom zusätzlichen Vorliegen eines Ausgangssignals der Ratenvergleichereinheit (127) abhängt.

9. Zweikammer-Herzschrittmacher nach einem der Ansprüche 4 bis 8, **dadurch gekennzeichnet, daß** die Logikstufe ein UND-Gatter (123) aufweist, mit dessen Eingängen der Ausgang der Zählmittel (117) direkt und der Ausgang der Testeinrichtung (119, 110, 120, 121) mindestens mittelbar verbunden sind.

10. Zweikammer-Herzschrittmacher nach Anspruch 8 und 9, **dadurch gekennzeichnet, daß** die Logikstufe ein dem UND-Gatter (123) vorgeschaltetes ODER-Gatter (122) aufweist, mit dessen Eingängen die Ausgänge der Testeinrichtung (119, 110, 120, 121) und der Ratenvergleichereinheit (127) verbunden sind.

11. Zweikammer-Herzschrittmacher nach einem der Ansprüche 4 bis 10, **dadurch gekennzeichnet, daß** die Stimulations-Steuereinheit (100A) Ratenbegrenzungsmittel (129) zur dynamischen Begrenzung der Rate der Ventrikelstimulation aufweist, die über mindestens einen Steuereingang mit dem Ausgang der Testeinrichtung (119, 110, 120, 121) und/oder dem Ausgang der Ratenvergleichereinheit (127) verbunden ist.

## Claims

1. A dual-chamber cardiac pacemaker (100) comprising
- atrium sensing means (EA, 107) for sensing electrical activity in the vestibule (A) and ventricle sensing means (EV, 2105) for sensing electrical activity in the ventricle (V) of a heart (H),
- a timer (104) for detecting the time of the occurrence of the respective electrical activity,
- ventricle stimulation means (106, EV) for producing stimulation pulses and outputting same to the ventricle, and
- a stimulation control unit (100A) for time control of production and output of the stimulation means, which has switching means (124) for switching over from an asynchronous to a vestibule-synchronous mode, criterion memory means (113, 116) for the storage of at least one switching-over criterion for mode switching-over and a processing unit (109, 111, 112, 114, 115) for processing signals from the atrium sensing means and the ventricle sensing means and the timer for checking fulfilment of the switching-over criterion,
**characterised in that**
- the criterion memory means has a signal sequence memory (113, 116) for storage of a predetermined selection of signal sequence patterns reflecting electrical activity of the heart and the processing unit has comparison means (112, 115) for comparison of the electrical activity detected by the atrium sensing means and the ventricle sensing means in a plurality of successive cardiac cycles to the stored signal sequence patterns, and
- the stimulation control unit further has counter means (117) connected to the comparison means on the input side for counting the number of cardiac cycles in which identity of the detected electrical activity with one of the pre-stored signal sequence patterns was detected, within a predetermined total number of successive cardiac cycles, the counting means being connected indirectly to a control input of the switching means.

2. A dual-chamber cardiac pacemaker according to claim 1 **characterised in that** at least a part of the pre-stored signal sequence patterns includes a predetermined succession of signals from the atrium sensing means (107) and the ventricle sensing means (105) or ventricle stimulation means (106) and a time interval criterion which concerns the spacing between two atrially sensed activities or an atrially sensed activity in relation to a ventricular activity.

3. A dual-chamber cardiac pacemaker according to claim 2 **characterised in that** the following signal sequence patterns are stored:
| | | |
|---|---|---|
| As-V-As | wherein As-As | > 1.000 ms |
| V-As-As-V | wherein As-As | > 1.000 ms |
| V-V-As | wherein V-As | > 1.000 ms |
| V-V | without interposed As, | |
wherein: As = atrially detected event and V = ventricularly detected or stimulated event.

4. A dual-chamber cardiac pacemaker according to one of the preceding claims **characterised in that** the stimulation control unit (100A) has a test device (119, 110, 120, 121) for carrying out and evaluating an active test for the presence of retrograde transfer, whose output is connected to an input of a logic stage (122, 123, 126, 128) connected between the counter means (117) and the switching means (124), so that activation of the switching means upon application of an output signal of the counter means depends on the additional presence of an output signal from the test device, representing a positive test result.

5. A dual-chamber cardiac pacemaker according to claim 4 **characterised in that** the test device has modulation means (119) for a predetermined modulation of the time spacing of successive ventricle stimuli, VA-detection means (110, 120) for detecting and storing a plurality of VA-time intervals between a respective ventricle stimulus and a subsequent atrial activity and VA-evaluation means (121) for comparative evaluation of the VA-time intervals as a reaction to modulation of the time spacing of the ventricle stimuli.

6. A dual-chamber cardiac pacemaker according to claim 5 **characterised in that** the modulation means (119) are adapted for an increase or reduction in length of the time spacings by a predetermined amount, in particular by a post-ventricular atrial blanking time and an additional increment, and the VA-evaluation means (121) output an output signal only when at least three successive VA-time intervals were constant after modulated ventricle stimuli.

7. A dual-chamber cardiac pacemaker according to one of the preceding claims **characterised in that** the criterion memory means (113, 116) are adapted for the storage of at least one rate limit value concerning the time intervals between successive atrial activities, in particular two rate limit values, and the processing unit has a calculation unit (109) for ascertaining the current atrial time intervals or atrial rate and a rate comparison unit (112, 117) for comparison thereof with the stored rate threshold value.

8. A dual-chamber cardiac pacemaker according to claim 7 and one of claims 4 to 6 **characterised in that** the output of the rate comparison unit (127) is connected to an input of the logic stage (122, 123, 126, 128) in such a way that activation of the switching means (124) upon application of an output signal from the counter means (117) depends on the additional presence of an output signal from the rate comparison unit (127).

9. A dual-chamber cardiac pacemaker according to one of claims 4 to 8 **characterised in that** the logic stage has an AND-gate (23), to the inputs of which the output of the counting means (117) is connected directly and the output of the test device (119, 110, 120, 121) is connected at least indirectly.

10. A dual-chamber cardiac pacemaker according to claims 8 and 9 **characterised in that** the logic stage has an OR-gate (122) which is connected on the input side of the AND-gate (123) and to the inputs of which are connected the outputs of the test device (119, 110, 120, 121) and the rate comparison unit (127).

11. A dual-chamber cardiac pacemaker according to one of claims 4 to 10 **characterised in that** the stimulation control unit (100A) has rate limiting means (129) for dynamically limiting the rate of ventricle stimulation, which is connected by way of at least one control input to the output of the test device (119, 110, 120, 121) and/or the output of the rate comparison unit (127).

## Revendications

1. Stimulateur cardiaque double chambre (100), comprenant :
- des moyens de détection de l'atrium (EA, 107) pour détecter une activité électrique dans l'oreillette (A) et des moyens de détection du ventricule (EV, 2105) pour détecter une activité électrique dans le ventricule (V) d'un coeur (H) ;
- un générateur de temps (104) pour saisir l'instant de l'apparition de l'activité électrique respective ;
- des moyens de stimulation du ventricule (106, EV) pour générer des impulsions de stimulation et les délivrer au ventricule ; et
- une unité de commande de stimulation (100A) pour commander dans le temps la génération et la sortie des impulsions de stimulation, qui présente des moyens de commutation (124) pour commuter d'un mode d'exploitation asynchrone à un mode d'exploitation synchrone avec l'oreillette, des moyens de mémoire de critères (113, 116) pour mémoriser au moins un critère de commutation pour la commutation de mode d'exploitation, et une unité de traitement (109, 111, 112, 114, 115) pour traiter des signaux provenant des moyens de détection de l'atrium et des moyens de détection du ventricule et du générateur de temps pour vérifier que le critère de commutation est satisfait ;
**caractérisé en ce que**
- les moyens de mémoire de critères présentent une mémoire de suite de signaux (113, 116) pour mémoriser une sélection prédéterminée de séquences de signaux reflétant une activité électrique du coeur, et l'unité de traitement présente des moyens de comparateur (112, 115) pour comparer l'activité électrique saisie par les moyens de détection de l'atrium et les moyens de détection du ventricule dans plusieurs cycles cardiaques consécutifs aux séquences de signaux mémorisées ; et
- l'unité de commande de stimulation présente de plus des moyens de compteur (117), reliés en entrée aux moyens de comparateur, pour compter le nombre de cycles cardiaques dans lesquels la coïncidence de l'activité électrique saisie avec l'une des séquences de signaux mémorisées à l'avance a été constatée, dans un nombre total prédéterminé de cycles cardiaques consécutifs, les moyens de compteur étant reliés indirectement à une entrée de commande des moyens de commutation.

2. Stimulateur cardiaque double chambre selon la revendication 1, **caractérisé en ce qu'**au moins une partie des séquences de signaux mémorisées à l'avance comprend une suite prédéterminée de signaux provenant des moyens de détection de l'atrium (107) et des moyens de détection du ventricule (105) ou des moyens de stimulation du ventricule (106) et un critère d'intervalle de temps concernant l'espacement de deux activités détectées de façon atriale ou d'une activité détectée de façon atriale par rapport à une activité ventriculaire.

3. Stimulateur cardiaque double chambre selon la revendication 2, **caractérisé en ce que** les séquences de signaux suivantes sont mémorisées :
| | | |
|---|---|---|
| As-V-As | où As-As | > 1000 ms |
| V-As-As-V | où As-As | > 1000 ms |
| V-V-As | où V-As | > 1000 ms |
| V-V | sans As intercalé | |
où : As = événement perçu de façon atriale et V = événement perçu de façon ventriculaire ou stimulé.

4. Stimulateur cardiaque double chambre selon l'une quelconque des revendications précédentes, **caractérisé en ce que** l'unité de commande de stimulation (100A) présente un dispositif de test (119, 110, 120, 121) pour exécuter et évaluer un test actif quant à la présence d'un transfert rétrograde, dont la sortie est reliée à une entrée d'un étage logique (122, 123, 126, 128) placé entre les moyens de compteur (117) et les moyens de commutation (124), de sorte qu'une activation des moyens de commutation en cas de disponibilité d'un signal d'entrée des moyens de comptage dépend de la présence supplémentaire d'un signal de sortie représentant un résultat de test positif du dispositif de test.

5. Stimulateur cardiaque double chambre selon la revendication 4, **caractérisé en ce que** le dispositif de test présente des moyens de modulation (119) pour une modulation prédéterminée du laps de temps des stimuli du ventricule successifs, des moyens de saisie VA (110, 120) pour saisir et mémoriser une pluralité d'intervalles de temps VA respectivement entre un stimulus du ventricule et une activité atriale consécutive et des moyens d'évaluation VA (121) pour une évaluation comparative de l'intervalle de temps VA en réaction à la modulation du laps de temps des stimuli du ventricule.

6. Stimulateur cardiaque double chambre selon la revendication 5, **caractérisé en ce que** les moyens de modulation (119) sont réalisés pour prolonger ou réduire les laps de temps d'une quantité prédéterminée, en particulier d'un temps de suppression atrial post-ventriculaire et d'un incrément supplémentaire, et les moyens d'évaluation VA (121) ne délivrent un signal de sortie que lorsque au moins trois intervalles de temps VA consécutifs après les stimuli du ventricule modulés ont été constants.

7. Stimulateur cardiaque double chambre selon l'une quelconque des revendications précédentes, **caractérisé en ce que** les moyens de mémoire de critères (113, 116) sont réalisés pour mémoriser au moins une valeur limite de fréquence concernant les intervalles de temps entre des activités atriales consécutives, en particulier deux valeurs de limite de fréquence et l'unité de traitement présente une unité de calcul (109) pour déterminer les intervalles de temps atriaux actuels ou la fréquence atriale et une unité de comparateur de fréquences (112, 127) pour les comparer à la valeur de seuil de fréquence mémorisée.

8. Stimulateur cardiaque double chambre selon la revendication 7 et l'une quelconque des revendications 4 à 6, **caractérisé en ce que** la sortie de l'unité de comparateur de fréquences (127) est reliée à une entrée de l'étage logique (122, 123, 126, 128) de telle sorte qu'une activation des moyens de commutation (124) dépend en cas de disponibilité d'un signal de sortie des moyens de compteur (117) de la présence supplémentaire d'un signal de sortie de l'unité de comparateur de fréquences (127).

9. Stimulateur cardiaque double chambre selon l'une quelconque des revendications 4 à 8, **caractérisé en ce que** l'étage logique présente une porte ET (123) aux entrées de laquelle la sortie des moyens de comptage (117) est reliée directement et la sortie du dispositif de test (119, 110, 120, 121) est reliée au moins indirectement.

10. Stimulateur cardiaque double chambre selon la revendication 8 ou 9, **caractérisé en ce que** l'étage logique présente une porte OU (122) placée en amont de la porte ET (123) aux entrées de laquelle sont reliées les sorties du dispositif de test (119, 110, 120, 121) et de l'unité de comparateur de fréquences (127).

11. Stimulateur cardiaque double chambre selon l'une quelconque des revendications 4 à 10, **caractérisé en ce que** l'unité de commande de stimulation (100A) présente des moyens de limitation de fréquence (129) pour une limitation dynamique de la fréquence de la stimulation du ventricule qui sont reliés par l'intermédiaire d'au moins une entrée de commande à la sortie du dispositif de test (119, 110, 120, 121) et/ou à la sortie de l'unité de comparateur de fréquences (127).
